# EUROPEAN PATENT APPLICATION

(11) **EP 4 597 103 A1**
(43) Date of publication of application: **06.08.2025**
(21) Application number: 23872581.6
(22) Date of filing: 22.09.2023
(51) Int. Cl.: G01N 33/50, C12N 5/079, C12Q 1/02, G01N 33/15, G01N 33/53, G01N 33/536

(54) **METHOD FOR ASSESSING COAGULATION INHIBITION ACTIVITY OR COAGULATION PROMOTION ACTIVITY AGAINST COAGULATIVE PROTEIN**

(30) Priority: 27.09.2022 JP 2022153259
(71) Applicant: Kaneka Corporation, Osaka-shi, Osaka 530-8288 (JP)
(72) Inventor: NISHISHITA Naoki, Kobe-shi, Hyogo 650-0047 (JP)
(74) Representative: Hoffmann Eitle
(86) International application number: PCT/JP2023/035564
(87) International publication number: WO 2024/071361

(57) **Abstract**

The objective of the present invention is to provide a method for screening for a substance having activity of inhibiting or promoting aggregation of aggregating proteins. More specifically, the present invention relates to a method for evaluating activity of a test substance for inhibiting or promoting aggregation of aggregating proteins comprising: a step of culturing cells in the presence of aggregating proteins labeled with a label and a test substance; and a step of quantifying the aggregated and/or deposited aggregating proteins on the cell surface and/or in the cells in the cell culture product with the use of the label as the indicator.

## Description

### Technical Field

The present invention relates to, for example, a method for evaluating activity of inhibiting or promoting aggregation of aggregating proteins used for screening for a substance having activity of inhibiting or promoting aggregation of aggregating proteins.

### Background Art

Alzheimer's disease (it is often abbreviated to "AD" herein) is a type of irreversible progressive central neurological disease associated with symptoms, such as cognitive impairment (dementia), behavioral disorders, or personality change. While the number of patients with dementia is deduced to be 50,000,000 or greater in 2019 all over the world, approximately 70% of such patients are considered to be patients with AD, and an incidence thereof is increasing. Because of increased medical expenses or problems of nursing care resulting from an increasing number of AD patients, economical or emotional burdens are imposed on the nations or persons involved in patients, and, accordingly, such problem is a serious issue of concern of the society in recent years.

AD is developed as follows. Hydrophobic peptides, amyloid β proteins (often referred to as "Aβ" herein), are aggregated and accumulated in the patient's brain, microtubule-binding proteins, tau proteins, are hyperphosphorylated to become fibrotic, neurons are destroyed, and the brain is then shrunk (Non-Patent Documents 1 to 3).

On the basis of the mechanism of AD development, a technique of evaluating effects of a test substance added *in vitro* for inhibiting Aβ aggregation and screening for a candidate compound for treatment of AD has been developed. For example, a method of microliter-scale high throughput screening (which is abbreviated to "MSHTS" herein) of an amyloid β protein aggregation inhibitor using a quantum dot nanoprobe is a cell-free assay-based screening technique that can search for a candidate compound having effects of inhibiting amyloid β protein aggregation in a PBS solvent (Non-Patent Document 4).

As an example of MSHTS, for example, Patent Document 1 discloses a method, an apparatus, and a program for evaluating amyloid formation. Specifically, Patent Document 1 discloses a method for determining inhibitory activity of a test substance on amyloid formation comprising: a step of aggregation comprising allowing an amyloid forming protein, such as an amyloid β protein, to react with a fluorescent probe (e.g., a probe comprising a quantum dot as a fluorescent dye or a quantum dot) capable of binding to an amyloid formed as a result of polymerization of the amyloid forming protein in an adequate buffer such as water or PBS in the presence or absence of the test substance; a step of photographing comprising photographing an image of a fluorescence of the aggregation product obtained in the step of aggregation; a step of calculating a standard deviation based on the luminance value of each pixel included in an area of interest in the fluorescent image photographed in the step of photographing; and a step of activity evaluation to determine that a test substance has inhibitory activity on amyloid formation when the standard deviation of the luminance in the presence of the test substance is smaller than the standard deviation of the luminance in the absence of the test substance as a result of comparison between the standard deviation of the luminance in the presence of the test substance and the standard deviation of the luminance in the absence of the test substance calculated in the step of calculating a standard deviation.

Furthermore, Patent Document 2 discloses a universal quantum dot nanoprobe used for evaluating amyloid aggregating properties of a protein or peptide and a method for evaluating an amyloid formation inhibitor using such quantum dot nanoprobe. Specifically, Patent Document 2 discloses a quantum dot nanoprobe comprising quantum dots bound to the N- or C-terminus of the amyloid forming peptide via cysteine.

However, inhibitory effects of a candidate compound selected by the cell-free assay system are not observed in the cell-based assay system (the test system using cells) in many occasions. This is considered to occur because the cell-free assay system does not necessarily reflect the *in vivo* environment.

### Prior Art Documents

### Patent Documents

Patent Document 1: WO 2020/138265
Patent Document 2: JP 2017-007990 A

### Non-Patent Documents

Non-Patent Document 1: Hardy J. and Selkoe D. J., 2002, Science, 297 (5580): 353-356
Non-Patent Document 2: Jack C. R. Jr., et al., 2010, Lancet Neurol., 9 (1): 119-128
Non-Patent Document 3: Akira Tamaoka, 2017, Proceedings of the Annual Meeting of the Japanese Research Group on Senile Dementia, Vol. 22, No. 3, pp. 19-23
Non-Patent Document 4: Ishigaki et al., 2013, PLOS ONE, 8 (8): e72992

### Summary of the Invention

Under the above circumstances, it is an object of the present invention to provide a screening method that can search for a candidate compound having effects of inhibiting or promoting aggregation of aggregating proteins, such as Aβ, in a cell-based assay (a test involving the use of cells) system.

The present inventors have conducted concentrated studies in order to solve the problems as described above. As a result, they found that, in a cell-based assay system, cell culture in the presence of aggregating proteins labeled with a label and a test substance, and quantification of aggregated and/or deposited aggregating proteins on the cell surface or in the cells in the cell culture product with the use of the label as the indicator would enable evaluation as to whether or not the test substance has activity of inhibiting or promoting aggregation of aggregating proteins. This has led to the completion of the present invention.

Specifically, the present invention includes the following.
[1] A method for evaluating activity of a test substance for inhibiting or promoting aggregation of aggregating proteins comprising:
   a step of culturing cells in the presence of aggregating proteins labeled with a label and a test substance; and
   a step of quantifying the aggregated and/or deposited aggregating proteins on the cell surface and/or in the cells in the cell culture product with the use of the label as the indicator.
[2] The method according to [1], wherein the cells are cells of the nervous system.
[3] The method according to [2], wherein the cells of the nervous system are a mixed cell system comprising neurons and glial cells.
[4] The method according to [3], wherein the neurons are provided by differentiation induction with the aid of autocrine amyloid β proteins generated in the process of differentiation from neural precursor cells into neurons.
[5] The method according to [1], wherein the aggregating proteins are amyloid β proteins.
[6] The method according to [1], wherein the label is an optical label.
[7] The method according to [6], wherein the optical label is a quantum dot.
[8] The method according to [1], wherein the step of quantification comprises:
   a step of photographing an image of the cells; and
   a step of calculating the number of the aggregating proteins, the area and/or the fluorescence intensity of a fluorescent region from the label as determined using fluorescence from the label as the indicator in the image obtained in the step of photographing an image as the aggregated and/or deposited aggregating proteins on the cell surface and/or in the cells.
[9] The method according to [1], wherein the quantification is performed while maintaining the cells alive with the elapse of time.
[10] A method for producing neurons comprising a step of inducing neural precursor cells to differentiate into neurons with the aid of autocrine amyloid β proteins generated in the process of differentiation from neural precursor cells into neurons.

The present description includes part or all of the content as disclosed in the description and/or drawings of Japanese Patent Application No. 2022-153259, which is a priority document of the present application.

### Brief Description of the Drawings

Figure 1 shows a chart demonstrating the results of evaluation concerning activity of *Hydrangea serrata var. thunbergii* and *Orthosiphon aristatus* for inhibiting aggregation in a cell-free system.
Figure 2 shows an image demonstrating the results of immunostaining of the cells of the nervous system prepared by the method according to the present invention.
Figure 3 shows charts each demonstrating the results of evaluation on activity of DMSO (solvent: negative control) and RA (rosmarinic acid: a substance exhibiting activity of inhibiting aggregation in a known cell-free evaluation system; positive control), *Hydrangea serrata var. thunbergii* and *Orthosiphon aristatus* for inhibiting aggregation in a cell-based system (the method according to the present invention).

### Embodiments of the Invention

Hereafter, the present invention is described in detail.

### 1. Method for evaluating activity of test substance for inhibiting or promoting aggregation of aggregating proteins

### 1-1. Outline

The first aspect of the present invention relates to a method for evaluating activity of a test substance for inhibiting or promoting aggregation of aggregating proteins. The method of the present aspect comprises: a step of culturing cells in the presence of aggregating proteins labeled with a label and a test substance; and a step of quantifying the aggregated and/or deposited aggregating proteins on the cell surface and/or in the cells in the cell culture product with the use of the label as the indicator. The method of the present aspect enables selection (screening) of a candidate substance exerting activity of inhibiting or promoting aggregation of aggregating proteins.

### 1-2. Definitions of terms

The following terms used herein are defined. Unless otherwise specified, the definitions of the terms below are used in common in other aspects of the present invention.

### (1) Test substance

Examples of test substances include nucleic acids, peptides, proteins, synthetic compounds, cell extracts, cell culture supernatants, plant extracts, and seaweed extracts.

### (2) Aggregating proteins

Aggregating proteins assemble to form an aggregate. Proteins that form an aggregate may be in a liquid or solid state, and types of aggregating proteins are not limited. Examples thereof include polyglutamic acids, disease-associated proteins, and autophagy-associated proteins.

Examples of disease-associated aggregating proteins include causal proteins of Alzheimer's disease, such as the amyloid β proteins and the tau protein (including the phosphorylated tau protein), a causal protein of Parkinson's disease, such as the α-synuclein protein, a causal protein of transmissible spongiform encephalopathies (including Creutzfeldt-Jakob disease, bovine spongiform encephalopathies, and prion disease), such as the prion protein, a causal protein of Huntington's disease, such as the huntingtin protein, a causal protein of type II diabetes, such as the amylin protein, a causal protein of arterial sclerosis (including cerebral infarction, pulmonary infarction, and myocardial infarction), such as the apolipoprotein A1 (APOA1 protein), a causal protein of articular rheumatism, such as the serum amyloid A protein, a causal protein of systemic AL amyloidosis, such as the immunoglobulin light chain, a causal protein of dialysis amyloidosis, such as the β2 microglobulin, and a causal protein of amyotrophic lateral sclerosis, such as the TDP-43 protein. In particular, the amyloid β proteins, such as the amyloid β40 protein, the amyloid β42 protein, the amyloid β43 protein, and the amyloid β38 protein, are preferable.

Examples of autophagy-associated proteins include the ubiquitin-like proteins, such as Atg-8 and Atg-12.

Aggregating proteins may be naturally-occurring proteins existing in nature, modified proteins derived from naturally-occurring proteins by artificial variation or modification, or artificial proteins comprising artificially designed amino acid sequences.

### (3) Aggregate

The term "aggregate" used herein refers to an assembly of two or more aggregating proteins. A so-called "protein complex" is within the scope of the "aggregate" herein.

An aggregate may be a homoaggregate consisting of a single type of proteins or a heteroaggregate consisting of different types of proteins.

### (4) Deposition

When aggregating proteins are "deposited" on the cell surface and/or in the cells herein, aggregating proteins are fixed on the cell surface and/or in the cells. The cells comprising aggregating proteins deposited on the surfaces thereof would experience lowered cell motility or cell death.

### (5) Cell

The target "cell" in the present invention is a cell that produces and/or secretes aggregating proteins.

A cell may be derived from a multicellular organism. An animal-derived cell is preferable, and a mammalian-derived cell is more preferable. Examples of animals include rodents, such as mice, rats, hamsters, and guinea pigs, livestock or pet animals, such as dogs, cats, rabbits, cows, horses, sheep, and goats, and primates, such as humans, rhesus monkeys, gorillas, and chimpanzees. A human-derived cell is particularly preferable.

A cell type is not limited. Examples thereof include a body-tissue-derived cell, a cell derived from a body-tissue-derived cell, a stem cell, a cell differentiated from a stem cell, and a precursor cell thereof.

The term "body tissue" refers to various tissue constituting the body of an organism. Examples thereof include epithelial tissue, connective tissue, muscle tissue, and nerve tissue.

The term "stem cell" refers to a cell having the potential to differentiate into different types of cells and the potential to self-renew. Examples thereof include an adult stem cell and a pluripotent stem cell.

The "adult stem cell" is a stem cell that exists in various tissue in an adult, the terminal differentiation thereof is incomplete, and such stem cell has a certain degree of pluripotency. An adult stem cell is also referred to as a somatic stem cell or tissue stem cell. Examples thereof include a mesenchymal stem cell, a neural stem cell, an intestinal epithelial stem cell, a hematopoietic stem cell, a hair follicle stem cell, a pigment stem cell, and a cancer stem cell.

The term "pluripotent stem cell" refers to a cell having a pluripotency to differentiate into all types of cells constituting an organism and capable of indefinitely growing while maintaining the pluripotency in *in vitro* culture under adequate conditions. Examples thereof include an embryonic stem (ES) cell, an embryonic germ stem cell, a germ stem cell, and an induced pluripotent stem (iPS) cell.

In the present invention, for example, cells of the nervous system, such as a mixed cell system comprising neurons and glial cells, are preferable. In the mixed cell system comprising neurons and glial cells, for example, neurons account for 10% to 70%, glial cells account for 30% to 80%, and other cells account for 20% or less of the entire system, and, preferably, neurons account for 40% to 50%, glial cells account for 45% to 55%, and other cells account for 5% or less of the entire system.

In the mixed cell system comprising neurons and glial cells, neurons are preferably provided by differentiation induction with the aid of autocrine amyloid β proteins generated in the process of differentiation from neural precursor cells into neurons. As described in the method for producing neurons according to the second aspect described below, cells in the process of differentiation or neurons differentiated at an early stage secrete amyloid β proteins during such process of differentiation. As a result, remaining neural precursor cells are induced to differentiate into neurons.

In addition, neurons further induced to differentiate from the neural precursor cells that have been induced to differentiate from iPS cells derived from an Alzheimer's disease (AD) patient can be used as cells that produce and secrete amyloid β proteins, such as amyloid β42 proteins.

### (6) Medium

In the present invention, a "medium" is a liquid or solid substance prepared to culture cells. In principle, a medium comprises the minimal amount or more of ingredients essential for growth and/or maintenance of cells. A medium may be a basal medium or a special cell culture medium.

A "basal medium" is a medium that constitutes a base of a medium for various animal cells. Culture can be performed in a basal medium, or various culture additives can be added to a basal medium to prepare a medium specific to any of various cells in accordance with purposes (i.e., a special cell culture medium). Examples of basal media that can be used include, but are not particularly limited to, Neurobasal^{®} medium, BME medium, BGJb medium, CMRL1066 medium, Glasgow MEM medium, Improved MEM Zinc Option medium, Iscove's Modified Dulbecco's (IMDM) medium, Medium 199 medium, Eagle MEM medium, αMEM medium, Dulbecco's Modified Eagle's (DMEM) medium, Ham's F10 medium, Ham's F12 medium, RPMI 1640 medium, Fischer's medium, and a medium mixture of any thereof (e.g., DMEM/F12 medium). In addition, media that are used for human iPS cell or human ES cell culture can be preferably used.

A "special cell culture medium" is a medium optimized for culture of special cells with the addition of various supplements to the basal medium as described above, or a medium prepared to be capable of inducing cells to differentiate into particular cells. Examples include culture media for cells of the nervous system commercialized by various manufacturers. A specific example is "M medium," which is obtained by adding, as supplements, NGF2.5S and BDNF to a nerve cell culture medium (Sumitomo Bakelite Co., Ltd.) that has been prepared by adding a culture supernatant of primary astroglia cells cultured on a nutritive medium and serum albumin to a basal medium prepared by adding insulin and transferrin to DMEM/F12 (5:5). A culture medium for pluripotent stem cells, such as human iPS cells or human ES cells, is also within the scope of the special cell culture medium.

A medium may or may not contain serum (i.e., a serum-free medium).

### (7) Labeling (Label)

The term "labeling" used herein refers to modification of a target substance to identify the target substance. The target substance can be detected or selected easily with certainty by labeling.

Labeling is performed in accordance with a type of a target substance. Since an aggregating protein is to be detected herein, the type of the target substance is a protein. Accordingly, any means of labeling that can directly or indirectly label a protein is employed. Examples of direct labeling include a method of labeling comprising binding a label substance to an aggregating protein and a method of labeling comprising expressing an aggregating protein as a fusion protein thereof with a label peptide. An example of indirect labeling is a method of labeling an aggregating protein with an antibody that specifically recognizes and binds to an aggregating protein or an active fragment thereof directly or indirectly via a secondary antibody. A method of binding a label substance is preferable.

A label substance used for protein labeling is not limited, and an example thereof is an optical label.

The term "optical label" used herein refers to a label with a fluorescent, luminescent, or other substance that emits light, such as visible light, near infrared light, or near-ultraviolet light.

A "fluorescent substance" is a substance that forms an excited state upon absorption of an excited light at a particular wavelength and emits fluorescence when it returns to the ground state. Fluorescent substances encompass a fluorescent dye and a fluorescent protein.

Examples of fluorescent dyes include quantum dot, FITC, Texas, Texas Red^{®}, Alexa Flour 405, Alexa Flour 488, Alexa Flour 647, Alexa Flour 700, Pacific Blue, DyLight 405, DyLight 550, DyLight 650, phycoerythrin-cyanin 5 (PE-Cy5), phycoerythrin-cyanin 7 (PE-Cy7), phycoerythrin (PE), peridinin chlorophyll protein (PerCP), peridinin chlorophyll protein-cyanin 5.5 (PerCP-Cy5.5), cy3, cy5, cy7, FAM, HEX, VIC^{®}, JOE, ROX, TET, Bodipy493, NBD, TAMRA, Quasar^{®} 670, Quasar^{®} 705, Allophycocyanin (APC), congo red, thioflavin T, thioflavin S, Fluorescamine or a derivative thereof, fluorescein or a derivative thereof, azo compound, rhodamine or a derivative thereof, coumarin or a derivative thereof, pyrene or a derivative thereof, and cyanine or a derivative thereof, with a quantum dot being preferable.

A "quantum dot" (which is often referred to as "QD" herein) is a nanoscale semiconductor crystal having quantum mechanics-based optical properties and emitting visible light and fluoresce in a near-infrared region. In general, a quantum dot is 2 nm to 10 nm in diameter and is composed of approximately 10 to 50 atoms. A quantum dot is excellent in properties, such that a large number of fluorescent colors are obtained depending on particle diameters, and fluorescence fading is less likely to occur. Accordingly, application thereof is advancing as a biosensing material and a cell or animal imaging material. Examples of quantum dots include semiconductor quantum dots, and specific examples thereof include core/shell CdSe/ZnS quantum dots, such as Qdot^{®} 525, Qdot545, Qdot565, Qdot585, Qdot605, Qdot655, Qdot705, and Qdot800 (Thermo Fisher Scientific).

An example of a "fluorescent protein" is GFP.

### 1-3. Method

Hereafter, the steps of the method according to the present aspect are described.

### 1-3-1. Step of culture

The "step of culture" comprises culturing (incubating) cells in the presence of aggregating proteins labeled with a label and a test substance. In this step, culture is performed to aggregate and/or deposit the aggregating proteins labeled with a label on the cell surface and/or in the cells.

For example, a solution containing labeled aggregating proteins diluted with a medium or the like and a solution containing a test substance diluted with a medium or the like are added to cells cultured in a culture vessel (e.g., a plate, such as a 96-well microplate). Alternatively, a solution containing unlabeled aggregating proteins diluted with a medium or the like may be added. Such solutions can be added simultaneously or continuously. The order of addition is not limited.

In a culture vessel, the cell seeding density (the cell density at the time of initiation of culture) in a solution (medium) can be adequately adjusted. For example, the density is 10,000 to 1,000,000 cells/ml, and the density is preferably 20,000 to 50,000 cells/ml.

In a culture vessel, the concentration of the aggregating proteins and that of the test substance in a solution (medium) may be as described below. For example, the final concentration of the labeled aggregating proteins may be 5 to 100 nM (preferably 10 to 50 nM), the final concentration of the test substance may be 1 to 100 ng/µl (preferably 10 to 50 ng/µl), and the final concentration of the unlabeled aggregating proteins may be 5 to 100 µM (preferably 10 to 50 µM).

Culture conditions are not particularly limited, provided that aggregating proteins can aggregate and/or deposit on the cell surface and/or in the cells. Temperature is, for example, 25°C to 39°C, 30°C to 38°C, or 35°C to 37°C. A duration is, for example, 30 minutes to 1,200 hours (50 days), 12 hours to 96 hours (4 days), 24 hours (1 day) to 72 hours (3 days), or 36 hours to 48 hours (2 days).

A mode of culture may be static culture or culture under medium-flowing conditions (flowing culture), with static culture being preferable.

Operation may be performed manually (culture may be performed manually by a person) or automatically using a program executed by an automated culture apparatus or the like.

### 1-3-2. Step of quantification

The "step of quantification" comprises quantifying aggregating proteins aggregated and/or deposited on the cell surface and/or in the cells in the cell culture product obtained in the step of culture using the label applied to the added aggregating proteins as the indicator after culture or with the elapse of time (e.g., every 30 minutes to 3 hours or every 1 hour to 2 hours) during culture while cells remain alive (culture remains continued). This step comprises quantitatively detecting aggregates/deposits of the aggregating proteins on the cell surface and/or in the cells.

When aggregating proteins are labeled with optical label substances such as quantum dots, for example, aggregates/deposits may be detected by fluorescent observation using a fluorescence microscope. A spotty fluorescent clot observed on the cell surface and/or in the cells indicates formation of aggregates/deposits. Subsequently, the fluorescence intensity is quantified, and, for example, the quantified fluorescence intensity is compared with the fluorescence intensity of the negative control to quantitatively detect the presence or absence of aggregates/deposits.

Specifically, the step of quantification can comprise: a step of photographing an image of the cells; and a step of calculating the number of the aggregating proteins, the area and/or the fluorescence intensity of a fluorescent region from the label as determined using fluorescence from the label, such as a quantum dot, as the indicator in the image obtained in the step of photographing an image as the aggregated and/or deposited aggregating proteins on the cell surface and/or in the cells.

When quantum dot-modified amyloid β proteins (e.g., amyloid β40 proteins) and unlabeled amyloid β proteins (e.g., amyloid β42 proteins) are added to the cells of the nervous system (amyloid β proteins (e.g., amyloid β42 proteins) are produced and/or secreted by neurons included in the nervous system), for example, the cell culture product is irradiated with a light, and aggregates/deposits including the amyloid β proteins as produced and/or secreted by the neurons, the unlabeled amyloid β proteins, and the quantum dot-modified amyloid β proteins on the cell surface and/or in the cells are visualized with the aid of the quantum dots. The procedure from image-obtaining (photographing) to analysis and evaluation of the number of aggregating proteins, the area and/or the fluorescence intensity of the fluorescent region by the visualization process described above can be performed using, for example, the live cell analysis system Incucyte^{®} (Sartorius).

When performing quantification with the elapse of time during culture (while culture remains continued), the conditions of Incucyte^{®} may be set as follows: Objective: 20×; scan type: Standard; Image channels: Phase, Red; Red Acquisition Times (ms): 400; Scan time: 1 shot/hr; and Picture number: 3 pictures/well, for example, on a 96-well microplate.

With the use of Incucyte^{®}, an image of each well is obtained, the image is then analyzed for a quantum dot-based fluorescence, and the number of particles in the fluorescence region in each well (equivalent to the number of aggregating proteins (the number of aggregates)), the area (µm²) and/or the fluorescence intensity (red calibration unit × µm²/well) of the fluorescence region is calculated. The number of aggregating proteins, the area and/or the fluorescence intensity of the fluorescence region are positively correlated with the amount of aggregated/deposited aggregating proteins. On the basis of the results of comparison of the number of aggregating proteins, the area and/or the fluorescence intensity of the fluorescence region with, for example, a negative control (e.g., in the absence of the test substance) and/or a positive control (e.g., in the presence of a known substance having activity of inhibiting or promoting aggregation), accordingly, whether or not the added test substance has activity of inhibiting or promoting aggregation of aggregating proteins is evaluated. When the number of aggregating proteins, the area and/or the fluorescence intensity of the fluorescence region are smaller than those of a negative control, for example, the added test substance can be determined to have activity of inhibiting aggregation. When the number of aggregating proteins, the area and/or the fluorescence intensity of the fluorescence region are larger than those of the negative control, the added test substance can be determined to have activity of promoting aggregation. When the number of aggregating proteins, the area and/or the fluorescence intensity of the fluorescence region are equivalent to or smaller than those of the positive control (e.g., in the presence of a known substance having activity of inhibiting aggregation), the added test substance can be determined to have activity of inhibiting aggregation. When the number of aggregating proteins, the area and/or the fluorescence intensity of the fluorescence region are equivalent to or larger than those of the positive control (e.g., in the presence of a known substance having activity of promoting aggregation), the added test substance can be determined to have activity of promoting aggregation.

### 1-4. Effect

The method of the present aspect is expected to show applicability as a tool for searching for a medicine useful for treatment or prevention of various diseases caused by aggregating proteins, including amyloidosis such as AD and Parkinson's disease in a state more similar to the *in vivo* environment.

### 2. Method for producing neurons

### 2-1. Outline

The second aspect of the present invention relates to a method for producing neurons comprising a step of inducing neural precursor cells to differentiate into neurons with the aid of autocrine amyloid β proteins generated in the process of differentiation from neural precursor cells into neurons. The method of the present aspect involves the use of the amyloid β proteins autosecreted by the cells for induction of differentiation. Thus, neurons can be efficiently prepared while reducing the amount of externally added differentiation inducers.

Furthermore, the neurons produced by the method of the present aspect can be preferably used in the method according to the first aspect of the present invention.

### 2-2. Method

Hereafter, the steps of the method according to the present aspect are described.

### 2-2-1. Step of preparing neural precursor cells

The method of the present aspect may comprise, as an optional step, a step of preparing neural precursor cells used for induction of differentiation by culture (incubation).

A form of a culture vessel used in the step is not particularly limited. For example, a culture vessel in the form of a dish, flask, well, bag, or spinner flask may be used.

A mode of culture may be static culture or culture under medium-flowing conditions (flowing culture), with static culture being preferable.

The neural precursor cell seeding density (the cell density at the time of initiation of culture) in a culture medium can be adequately adjusted. For example, the density is 0.01×10⁵ cells/ml or higher, more preferably 0.1×10⁵ cells/ml or higher, and more preferably 1×10⁵ cells/ml or higher, 20×10⁵ cells/ml or lower, and more preferably 10×10⁵ cells/ml or lower.

In this step, neural precursor cells are cultured in a neural precursor cell culture medium (e.g., STEMdiff Neural Progenitor Medium (NPM) (STEMCELL)). The medium may be supplemented with a ROCK inhibitor.

A ROCK inhibitor is defined as a substance that inhibits kinase activity of Rhoassociated protein kinase (Rho-kinase (ROCK)). Examples thereof include Y-27632 (4-[(1R)-1-aminoethyl]-N-pyridin-4-ylcyclohexane-1-carboxamide or a salt thereof (e.g., dihydrochloride)), H-1152 ((S)-(+)-2-methyl-1-[(4-methyl-5-isoquinolinyl)sulfonyl]-hexahydro-1H-1,4-diazepine or a salt thereof (e.g., dihydrochloride)), Fasudil/HA1077 (1-(5-isoquinoline sulfonyl)homopiperazine or a salt thereof (e.g., dihydrochloride)), Wf-536 ((+)-(R)-4-(1-aminoethyl)-N-(4-pyridyl)benzamide monohydrochloride), Y39983 (4-[(1R)-1-aminoethyl]-N-1H-pyrrolo[2,3-b]pyridin-4-ylbenzamide dihydrochloride), SLx-2119 (2-[3-[4-(1H-indazol-5-ylamino)-2-quinazolinyl]phenoxy]-N-(1-methylethyl)-acetamide), azabenzimidazole-aminofurazans, DE-104, XD-4000, HMN-1152, 4-(1-aminoalkyl)-N-(4-pyridyl)cyclohexane-carboxamides, Rhostatin, BA-210, BA-207, BA-215, BA-285, BA-1037, Ki-23095, and VAS-012.

The concentration of a ROCK inhibitor in the medium is, for example, 0.1 to 50 nM, and preferably 1 to 5 nM.

Conditions, such as culture temperature and a culture period, are not particularly limited. Culture temperature is, for example, 25°C to 39°C, 30°C to 38°C, or 35°C to 37°C. A culture period is, for example, 30 minutes to 72 hours (3 days), 12 hours to 48 hours (2 days), or 20 hours to 36 hours.

### 2-2-2. Step of inducing neural precursor cells to differentiate into neurons

This step comprises inducing neural precursor cells to differentiate into neurons.

In this step, neural precursor cells are cultured in a nerve cell culture medium (e.g., Neurobasal Medium, minus phenol red (Nb) (Gibco) supplemented with B-27 supplement (Gibco) and Penicillin-Streptomycin Mixed Solution (P/S) (Nacalai Tesque); the NbM medium) and induced to differentiate into a mixed cell system comprising neurons and glial cells. The medium is further supplemented with the hedgehog protein (e.g., Recombinant human Sonic Hedgehog (SHH, Veritas Corporation) as an essential ingredient.

A differentiation inducer, such as an amyloid β protein, may be also added externally to the medium. The concentration of the amyloid β protein externally added to the medium is, for example, 1 to 1000 µM, and preferably 1 µM to 100 µM.

In a preferable embodiment of the present step, the amyloid β proteins and CCL2 autosecreted by neurons during the process of differentiation from neural precursor cells into neurons are accumulated in the culture supernatant and used for induction of differentiation. After the hedgehog protein is added at the beginning of the present step, more specifically, the total volume of the medium is not exchanged. During the process of differentiation, the amyloid β proteins or CCL2 secreted by cells in the process of differentiation, neurons differentiated at an early stage, astrocytes, and the like are accumulated at given concentrations in the culture supernatant. Thus, neural precursor cells can be induced to differentiate into a mixed cell system comprising neurons and glial cells with the exclusive addition of the hedgehog protein as an externally added differentiation inducer.

**In** this step, neural precursor cells recovered from the step of preparing neural precursor cells as described above may be seeded and cultured in another similar culture vessel. It is preferable that the neural precursor cells in the culture vessel after the step of preparing neural precursor cells as described above are cultured by performing medium exchange.

A mode of culture may be static culture or culture under medium-flowing conditions (flowing culture or agitation culture), with static culture being preferable.

Conditions, such as culture temperature and a culture period, are not particularly limited. Culture temperature is, for example, 25°C to 39°C, 30°C to 38°C, or 35°C to 37°C. A culture period is, for example, 1 day to 21 days, 5 days to 16 days, or 9 days to 12 days.

A medium is added and/or a part of a medium is exchanged at an adequate frequency to lower the hedgehog protein concentration in the medium in a stepwise manner. Also, the autocrine amyloid β proteins generated in the process of differentiation from neural precursor cells into neurons and the like are accumulated in the supernatant. Thus, neural precursor cells are induced to differentiate into neurons. When culture is performed for 12 days, for example, the hedgehog protein concentration in the medium is, for example, 1 to 300 ng/µl, and preferably 10 to 100 ng/µl on Day 0 of culture; the hedgehog protein concentration in the medium is, for example, 0.5 to 150 ng/µl, and preferably 5 to 50 ng/µl on Day 2 of culture; the hedgehog protein concentration in the medium is, for example, 0.01 to 75 ng/µl, and preferably 0.1 to 25 ng/µl on Day 6 of culture; and the hedgehog protein concentration in the medium is, for example, 0.01 to 2.5 ng/µl, and preferably 0.1 to 2.0 ng/µl on Day 9 of culture.

A medium may be added and/or a part of a medium may be exchanged at a frequency of, for example, at least once every 5 days, more preferably at least once every 4 days, further preferably at least once every 3 days, furthermore preferably at least once every 2 days, and the most preferably at least once a day.

In the mixed cell system comprising neurons and glial cells obtained in the present step, for example, neurons account for 10% to 70%, glial cells account for 30% to 80%, and other cells account for 20% or less of the entire system, and, preferably, neurons account for 40% to 50%, glial cells account for 45% to 55%, and other cells account for 5% or less of the entire system.

In the present step, whether or not neural precursor cells are induced to differentiate into neurons can be determined using the neural precursor cell marker and/or the nerve cell marker as the indicator. An example of a neural precursor cell marker is Nestin. An example of a nerve cell marker is MAP2.

The neural precursor cell marker and/or the nerve cell marker can be detected by any method of detection available in the art. An example of a method for detecting an expression marker is, but is not limited to, flow cytometry. When a cell that emits stronger fluorescence than a negative control (isotype control) is detected in flow cytometry performed with the use of a fluorescence-labeled antibody, such cell is determined to be "positive" for the marker. The rate of cells that are positive for the fluorescence-labeled antibody analyzed by flow cytometry may be referred to as the "positive rate." Any fluorescence-labeled antibodies known in the art can be used herein. Examples thereof include, but are not limited to, antibodies labeled with fluorescein isothiocyanate (FITC), phycoerythrin (PE), and allophycocyanin (APC).

The neural precursor cell marker and/or the nerve cell marker can also be detected by immunostaining. For example, cells in the culture vessel are allowed to react with the primary antibody and then with the fluorescence-labeled secondary antibody, and fluorescence-emitting cells are visually detected by fluorescence microscopy. For example, the rate of cells stained with the fluorescence-labeled secondary antibody relative to the cells stained with DAPI (a cell nuclear marker) can be determined as the positive rate. Any antibody known in the art can be used as the fluorescence-labeled secondary antibody. An example thereof is an antibody labeled with Alexa Fluor488, although the antibody is not limited thereto.

When the rate (proportion) of cells that are positive for the neural precursor cell marker is 10% or lower, and preferably 5% or lower, and/or the rate (proportion) of cells that are positive for the nerve cell marker is 70% or higher, and preferably 80% or higher, for example, it is possible to determine that the neurons are induced to differentiate from the neural precursor cells to a significant extent in the present step.

### 2-2-3. Step of recovery

The "step of recovery" comprises recovering cultured cells from the culture solution after the step of induction of differentiation, and the step of recovery is an optional step in the method of the present aspect.

"Recovery of cells" is a procedure of obtaining cells by separating cells from the culture solution. A method for recovering cells may be performed in accordance with a conventional method employed in the method of cell culture in the art, and a method is not particularly limited.

The mixed cell system comprising neurons and glial cells obtained in the method of the present aspect is obtained by adhesion culture, and many cells remain adhered to an external matrix, such as a culture vessel or culture support, after culture. In order to remove the culture solution from the culture vessel, accordingly, the vessel may be gently inclined to flush the liquid component out of the vessel after culture. Because the cells that have adhered to the external matrix remain in the culture vessel, the cells can be easily separated from the culture solution.

Thereafter, the surface of the cells adhered to the external matrix can be washed, according to need. As a wash solution, a buffer (including PBS buffer), physiological saline, or a medium may be used, although a wash solution is not limited thereto. After washing, the used wash solution may be removed in the same manner as in the case of the culture solution. The step of washing may be repeated a plurality of times.

Subsequently, a cell population that has adhered to the external matrix is detached from the external matrix. A method of detaching known in the art may be adopted. In general, detachment is performed by scraping, or using a detaching agent comprising, as an active ingredient, a protease, a chelating agent such as EDTA, a mixture of a detaching agent and a chelating agent, or the like.

Scraping is a method of detaching the cells adhered to the external matrix therefrom with the use of a scraper or the like by a mechanical means. It should be noted that cells are likely to be damaged by mechanical operation. When the recovered cells are further cultured, accordingly, a method of detaching comprising chemically destroying or decomposing the scaffold of the cells fixed on the external matrix to release the adhesion between the cells and the external matrix is preferable.

The method of detaching involves the use of a detaching agent and/or a chelating agent. A detaching agent is not limited. For example, commercially available detaching agents, such as Accutase^{®}, TrypLE^{™} Express Enzyme (Life Technologies), TrypLE^{™} Select Enzyme (Life Technologies), and Dispase^{®}, can be used, in addition to trypsin, collagenase, pronase, hyaluronidase, and elastase. Detaching agents may be used at concentration for a period of time in accordance with a conventional technique used for cell detachment or dispersion.

The supernatant containing the detaching agent is removed by centrifugation to recover the cells detached from the external matrix. The cells are thus recovered.

### 2-3. Effects

According to the method of the present aspect, neurons useful for transplantation or research can be prepared efficiently.

### Examples

Hereafter, the present invention is described in greater detail with reference to Examples, although the technical scope of the present invention is not limited to these Examples.

### (Reference Example 1) Preparation of extracts of various materials (test substances)

### (1) Hydrangea serrata var. thunbergii extract

*Hydrangea serrata var. thunbergii* (dry weight: 1.03 g) was grounded using a mill, 30 ml of 90% ethanol was added to approximately 1 g of the grounded material, and the *Hydrangea serrata var. thunbergii* extract was obtained by shaking extraction at 28°C and 150 rpm in the dark for 20 hours. The extract was subjected to centrifugation at 3,000 rpm for 20 minutes, cotton-plug filtration, further centrifugal concentration, and then lyophilization. The lyophilized product was dissolved in DMSO to prepare a 100 mg/ml *Hydrangea serrata var. thunbergii* extract, and the resultant was stored at -20°C before use.

### (2) Orthosiphon aristatus extract

*Orthosiphon aristatus* (dry weight: 1.01 g) was treated in the same manner as in the case of preparation of the *Hydrangea serrata var. thunbergii* extract above to prepare a 100 mg/ml *Orthosiphon aristatus* extract, and the resultant was stored at -20°C before use.

### (Reference Example 2) Evaluation of activity of inhibiting amyloid β aggregation in cell-free system

### (1) Preparation of amyloid β protein (Aβ) solution

To 5 mg of Aβ (human, 1-42) (Peptide Institute, Inc.), 5 ml of HFIP (1,1,1,3,3,3-hexafluoro-2-propanol) (FUJIFILM Wako Pure Chemical Corporation) was added to prepare a suspension, and the suspension was then allowed to stand at room temperature for 1 hour. Thereafter, the suspension was ultrasonicated at 25°C and 43 kHz for 10 minutes for monomerization, and the resultant was allowed to stand in a clean bench for 24 hours to vaporize HFIP. Thereafter, the resultant was dissolved in 1,071 µl of DMSO to obtain a 1 mM Aβ solution. The Aβ solution was dispensed into 1.5-ml tubes in an amount of 256 µl each and stored at -80°C before use.

The Aβ solution prepared herein is hereafter referred to as "Aβ42."

### (2) Preparation of quantum dot-modified amyloid β protein (QDAβ)

Aβ was modified with a quantum dot (QD) in the manner described below to prepare a quantum dot-modified amyloid β protein (QDAβ).

Qdot^{™}605 ITK^{™} amino (PEG) Quantum Dots (8 µM, 125 µl) were introduced into two 1.5-ml tubes, and the tubes were centrifuged at 10,000× g and 4°C for 1 minute. The supernatants thereof were transferred to centrifugal concentration tubes, and 4,500 µl of PBS was added thereto. Centrifugation was continued at 4°C and 3,800× g until the total amount in the two tubes was reduced to about 50 µl or less, and the filtrates were discarded. After PBS was filled, centrifugation was performed again until the amount was reduced to 50 µl. The QD solutions obtained were introduced into a tube to adjust the total amount to approximately 180 µl. 10 mM sulfo-EMCS (20 µl) was added, and the resultant was allowed to stand at room temperature for 1 hour to prepare QD-EMCS. After the preparation of QD-EMCS, 20 µl of 100 mM K-glutamic acid was added in order to inactivate an unreacted N-hydroxysuccinimide group contained in the solution, and the resultant was allowed to stand at room temperature for 10 minutes.

Two columns for desalting were prepared, approximately 800 µl of resin was introduced into each thereof, and centrifugation was performed at 1,000× g and 4°C for 1 minute. PBSE (300 µl) was loaded onto each column and centrifugation was performed at 1,000× g and 4°C for 1 minute. This procedure was performed two times to prepare desalting columns.

The QD-EMCS (110 µl) was allowed to impregnate into the center of each of two desalting columns, and 15 µl of PBSE was added as a stacker. After the columns were subjected to centrifugation at 1,000× g and 4°C for 2 minutes, filtrates (desalted QD-EMCS) of the two desalting columns were gathered together. To the desalted QD-EMCS obtained, 20 µl of 1.0 mM Cys-Aβ/DMSO was added and mixed therein, and the resulting mixture was then allowed to stand at room temperature for 1 hour. Thereafter, 20 µl of 100 mM 2-mercaptoethanol was added in order to inactivate an unreacted maleimide group contained in the solution, and the resultant was allowed to stand at room temperature for 10 minutes.

According to the operation as described above, the filtrate was transferred in an amount of 145 µl each to two centrifugal concentration tubes, 4,500 µl of water was added thereto, and the resultants were centrifuged at 3,800× g and 4°C for 17 minutes. The filtrates were discarded, and the obtained solutions were introduced into a tube to adjust the total amount of the obtained solutions to approximately 140 µl.

Water (300 µl) was introduced into a desalting column, centrifugation was performed at 1,000× g and 4°C for 1 minute, and this procedure was performed two times. The filtrate (70 µl) was allowed to infiltrate into the desalting column, and 15 µl of stacker water was added. Centrifugation was performed at 1,000× g and 4°C for 2 minutes to obtain QDAβ of interest.

### (3) Evaluation of activity of each material extract for inhibiting Aβ aggregation

The filtration filter device (Amicon Ultra-0.5 Ultracel-50 Membrane) was mounted on a 1.5-ml tube, and 400 µl of MilliQ water was added dropwise to the filtration filter device, and the tube was centrifuged at room temperature (15°C to 25°C) and 14,000× g for 10 minutes. The filtrate was discarded, 400 µl of MilliQ water was added again dropwise to the filter, and the tube was centrifuged at room temperature (15°C to 25°C) and 14,000× g for 10 minutes. The filtrate was discarded, 500 µl of an aqueous 0.1% Tween 20 solution was added dropwise to the filtration filter, and the resultant was allowed to stand at room temperature (15°C to 25°C) for 2 hours.

After blocking, the tube was centrifuged, the filtrate was discarded, and the tube was centrifuged at room temperature (15°C to 25°C) and 14,000× g for 10 minutes. The filtrate was discarded, 400 µl of MilliQ water was added dropwise to the filter, the tube was centrifuged at room temperature (15°C to 25°C) and 14,000× g for 10 minutes, the filtrate was discarded again, 400 µl of MilliQ water was added dropwise to the filter, the tube was centrifuged at room temperature and 14,000× g for 10 minutes, the filtrate was discarded, and the empty tube was centrifuged at room temperature (15°C to 25°C) and 14,000× g for 2 minutes.

The filtration filter device was mounted on another 1.5-ml tube, (i) 500 µl of PBS or (ii) 500 µl of a culture supernatant (the concentration of Aβ₄₂ contained in the culture supernatant is measured in advance using the human β amyloid ELISA Kit (FUJIFILM Wako Pure Chemical Corporation) obtained by culture of AD-iPS-derived cells of the nervous system in the NbM medium (prepared by diluting B27 (Life Technologies Corporation) to 50-fold with Neurobasal medium (without phenol red) (Life Technologies Corporation)) was added dropwise to the filtration filter, the tube was centrifuged at room temperature (15°C to 25°C) and 14,000× g for 30 minutes, and the filtrate was recovered. The QD-Aβ40 solution (final concentration: 50 nM), the Aβ42 solution (final concentration), and the material extract were added to the filtrate (PBS or the culture supernatant) to prepare reaction solutions. A plurality of reaction solutions each containing the material extract at the final concentration of 1500 µM, 300 µM, 60 µM, 12 µM, and 2.4 µM, respectively, were prepared.

The reaction solutions were incubated at 37°C for 24 hours, and the amount of Aβ aggregation was quantified at each of various concentrations of each material.

The amount of Aβ aggregation was measured with the use of the value of luminance SD as the indicator based on a positive correlation between the value of luminance SD calculated from the fluorescent image of the aggregate and the amount of Aβ aggregation. A fluorescent image was obtained by mounting a vessel (microwell plate) accommodating a reaction solution on an epifluorescence microscope equipped with a CCD (or CMOS) camera, applying an excitation light thereto, allowing fluorescence to emit from the reaction product, and photographing an image thereof using a CCD camera.

An excitation light may be adequately determined in accordance with properties of a quantum dot used. When Qdot 605 is used as a quantum dot, for example, the wavelength of the excitation light may be shorter than 580 nm, and it may be 532 to 552 nm.

In order to allow the emitted fluorescence to selectively transmit and photograph an image of the fluorescence, a band pass filter may be selected in accordance with the fluorescence emitted. When Qdot 605 is used as a quantum dot, for example, a band pass filter that can selectively transmit a light in a wavelength band from 594 nm to 646 nm can be used.

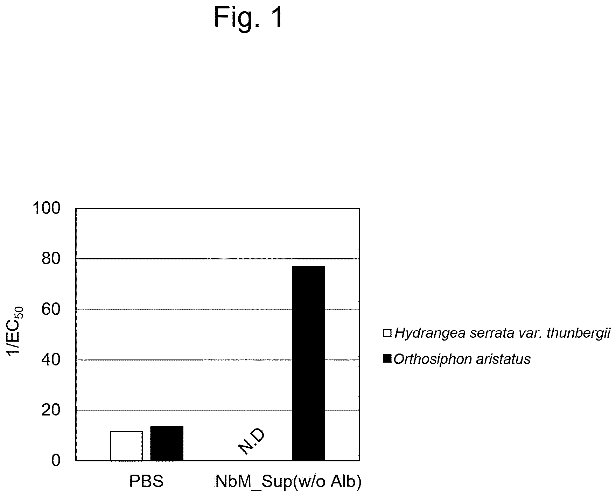

On the basis of the results, the concentration at which Aβ accounting for 50% of the whole would not aggregate (EC₅₀) was calculated for each material. When the calculated EC₅₀ is designated as the reciprocal, the value of interest would become 0 or more in the presence of activity of inhibiting aggregation. As inhibitory activity is increased, also, the value of interest would become larger. This facilitates comparison. Figure 1 shows 1/EC₅₀ of each material.

Figure 1 demonstrates that *Orthosiphon aristatus* exhibited activity of inhibiting Aβ aggregation in both evaluation systems using (i) PBS and (ii) the culture supernatant of AD-iPS-derived cells of the nervous system (NbM_Sup (w/o Alb)), while *Hydrangea serrata var. thunbergii* exhibited activity in the system using (i) PBS but did not exhibit activity in the system using (ii) the culture supernatant (N.D.).

### (Example 1) Preparation of cells of nervous system for evaluation

As the cells used for evaluation of activity of inhibiting Aβ aggregation according to an embodiment of the present invention, cells of the nervous system were prepared in the manner described below.
(1) Preparation of reagents and the like
   (i) NbM medium:
      Neurobasal Medium, minus phenol red (50 ml, Gibco), was supplemented with 1 ml of B-27 supplement (Gibco) and the Penicillin-Streptomycin Mixed Solution (Nacalai Tesque) to prepare the NbM medium.
   (ii) NbM medium supplemented with Y-27632:
      Y-27632 (5 mg, Nacalai Tesque) was dissolved in 1561 µl of PBS (stored at -30°C before use). This Y-27632 was diluted to 2000-fold and added to the above-described NbM medium to prepare the NbM medium supplemented with Y-27632.
   (iii) Matrigel-PBS:
      hESC-Qualified Matrigel (500 µl, Corning) was added to 25 ml of PBS and suspended therein to prepare matrigel-PBS.
   (iv) NbM medium supplemented with SHH:
      DMSO (100 µl) was added to 10 µg of Recombinant human Sonic Hedgehog (Veritas Corporation) to prepare an SHH solution. This SHH solution (10 µl) was added to 10 ml of the above-described NbM medium to prepare the NbM medium supplemented with SHH.
(2) Preparation of cells of nervous system for evaluation

The AD-iPS-derived neural precursor cells that had been cultured in advance were detached, centrifuged, washed, and suspended in the NbM medium supplemented with Y-27632 to prepare a suspension of 3×10⁵ cells/ml neural precursor cells. This suspension was fractionated at 100 µl/well on the 96-well flat bottom plate coated with matrigel-PBS in advance to seed the cells (3 × 10⁴ cells/well). The plate was allowed to stand and incubated at 37°C for 1 day.

### (Day 0)

The total volume of the medium was exchanged with the NbM medium supplemented with SHH (SHH concentration in well: 0.100 ng/µl),and incubation was continued. The day on which the procedure described above was performed was designated as Day 0, and the course of time of incubation thereafter was denoted as Day X.

### (Day 2)

A fresh NbM medium was added at 50 µl/well (SHH concentration: 0.067 ng/µl),and incubation was continued.

### (Day 6)

A half volume of the medium (approximately 60 µl/well) was removed while refraining from detaching the cells from the plate, a fresh NbM medium was then added at 70 µl/well, and incubation was continued.

### (Day 9)

A fresh NbM medium was added at 50 µl/well, and incubation was continued.

### (Day 12)

Preparation of the cells of the nervous system for evaluation was completed.

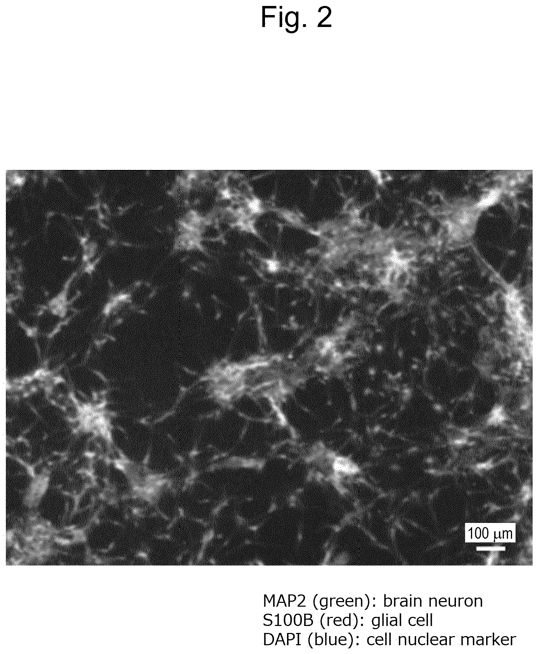

Figure 2 shows an image demonstrating the results of immunostaining of the cells on Day 12.

Figure 2 demonstrates that the cells of the nervous system for evaluation prepared by the method of the present invention made up a mixed cell system including neurons and glial cells that has conditions similar to those of the *in vivo* conditions. The rate of neurons (MAP2-positive cells), glial cells (S100B-positive cells), and unstained cells was examined by image analysis. As a result, the rate thereof was found to be 44.7%, 51.1%, and 4.2%, respectively. The rate of the unintended cells (unstained cells) was 5% or lower. This indicates that the mixed cell system was very good in terms of cell quality.

In this method, the total volume of the medium was not exchanged on Day 0 or later, Aβ autosecreted by the cells was accumulated to induce the cells to differentiate into neurons, and a mixed cell system including neurons and glial cells could be prepared with the exclusive use of SHH as a differentiation inducer that was externally added.

### (Example 2) Evaluation of activity of inhibiting amyloid β aggregation in cell-based assay

The Aβ42 solution and the QD-Aβ solution prepared in Reference Example 2 (1) and (2) were each diluted with a fresh NbM medium to prepare the adjusted Aβ42 solution (10.7 µM) and the adjusted QD-Aβ solution (64.3 nM). Also, the material extracts prepared in Reference Example 1 were each diluted with a fresh NbM medium to prepare material-containing solutions (300 ng/µl). In addition, DMSO diluted by the same factor as in the case of the material extract was used as a negative control, and rosmarinic acid (RA, final concentration: 50 µM, a substance exhibiting activity of inhibiting aggregation in a known cell-free evaluation system) was also used as a positive control.

The culture supernatant of the cells of the nervous system for evaluation prepared in Example 1 (96-well plate) was removed using a pipette, and the adjusted QD-Aβ solution as described above was fractionated at 70 µl/well carefully while refraining from detaching the cells. Subsequently, the material-containing solution (10 µl/well) as described above and the adjusted Aβ42 solution (70 µl/well) as described above were further injected thereinto (final concentration of QD-Aβ: 30 nM; final concentration of Aβ42: 10 µM). A gas permeable seal was applied to the plate (without a cap), the plate was mounted on the live cell analysis system Incucyte^{®} (Sartorius) set in an incubator at 37°C in the presence of a 5% CO₂, and observation was then performed for 48 hours.

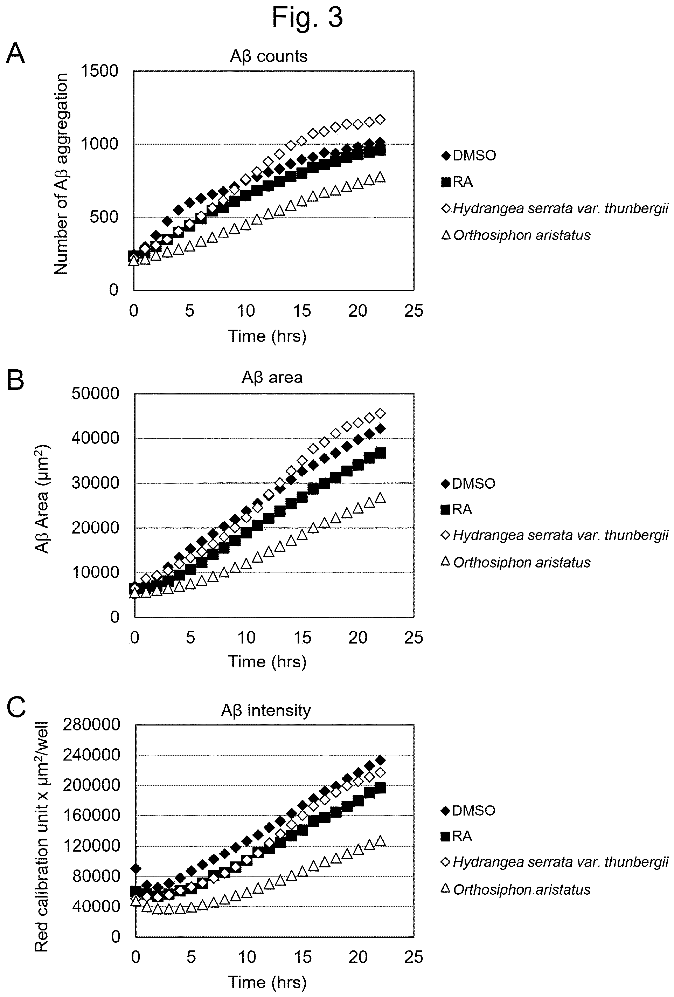

The conditions of Incucyte^{®} was set as follows.
Objective: 20 x
Scan type: Standard
Image channels: Phase, Red
Red Acquisition times (ms): 400
Scan time: 1 shot/hr
Picture number: 3 pictures/well
Figure 3 shows the results.

The plot line lower than that of the negative control DMSO on the Y axis indicates that the material extract has activity of inhibiting Aβ aggregation/deposition on the cells of the nervous system. A greater fall of the plot line indicates higher activity.

Specifically, the *Orthosiphon aristatus* extract exhibited high activity. In contrast, the *Hydrangea serrata var. thunbergii* extract did not show a difference from the negative control. That is, the *Hydrangea serrata var. thunbergii* extract had no activity of inhibiting Aβ aggregation/deposition on the cell surface or in the cells.

These results were highly correlated with the results attained by the cell-free evaluation method performed with the use of the culture supernatant of the AD-iPS-derived cells of the nervous system in Reference Example 2.

### Industrial Applicability

The present invention enables selection of a candidate substance that actually exerts activity of inhibiting or promoting aggregation of aggregating proteins from among candidate substances added to the culture product of disease model cells by quantification of the aggregated and/or deposited aggregating proteins. In particular, the present invention enables screening of a candidate compound for treatment of AD.

All publications, patents, and patent applications cited herein are incorporated herein by reference in their entirety.

## Claims

1. A method for evaluating activity of a test substance for inhibiting or promoting aggregation of aggregating proteins comprising:
a step of culturing cells in the presence of aggregating proteins labeled with a label and a test substance; and
a step of quantifying the aggregated and/or deposited aggregating proteins on the cell surface and/or in the cells in the cell culture product with the use of the label as the indicator.

2. The method according to claim 1, wherein the cells are cells of the nervous system.

3. The method according to claim 2, wherein the cells of the nervous system are a mixed cell system comprising neurons and glial cells.

4. The method according to claim 3, wherein the neurons are provided by differentiation induction with the aid of autocrine amyloid β proteins generated in the process of differentiation from neural precursor cells into neurons.

5. The method according to claim 1, wherein the aggregating proteins are amyloid β proteins.

6. The method according to claim 1, wherein the label is an optical label.

7. The method according to claim 6, wherein the optical label is a quantum dot.

8. The method according to claim 1, wherein the step of quantification comprises:
a step of photographing an image of the cells; and
a step of calculating the number of the aggregating proteins, the area and/or the fluorescence intensity of a fluorescent region from the label as determined using fluorescence from the label as the indicator in the image obtained in the step of photographing an image as the aggregated and/or deposited aggregating proteins on the cell surface and/or in the cells.

9. The method according to claim 1, wherein the quantification is performed while maintaining the cells alive with the elapse of time.

10. A method for producing neurons comprising a step of inducing neural precursor cells to differentiate into neurons with the aid of autocrine amyloid β proteins generated in the process of differentiation from neural precursor cells into neurons.
